# EUROPÄISCHE PATENTSCHRIFT

(11) **EP 3 192 476 B1**
(45) Veröffentlichungstag und Bekanntmachung des Hinweises auf die Patenterteilung: **26.07.2023**
(21) Anmeldenummer: 17150938.3
(22) Anmeldetag: 11.01.2017
(51) Int. Cl.: A61F 7/00

(54) **MEDIZINISCHES TEMPERIERGERÄT**
MEDICAL TEMPERATURE CONDITIONING APPARATUS
APPAREIL MÉDICAL DE RÉGULATION DE TEMPÉRATURE

(30) Priorität: 14.01.2016 DE 102016000345
(43) Veröffentlichungstag der Anmeldung: 19.07.2017
(73) Patentinhaber: Hilotherm Holding AG, 6317 Oberwil bei Zug (CH)
(72) Erfinder: Janisch, Klaus, 88316 Isny (DE)
(74) Vertreter: Wallinger, Michael

(56) Entgegenhaltungen:
- EP-A1- 1 820 480
- EP-A1- 2 929 862
- EP-B1- 2 965 026
- US-A1- 2005 028 551
- US-A1- 2014 277 302

## Beschreibung

Die Heilwirkung von Wärme- und Kältebehandlungen für den menschlichen und tierischen Körper ist schon seit langem bekannt. Während einfache Temperierungseinrichtungen, wie Eisbeutel oder Wärmeflaschen, als Hausmittel nach wie vor Verwendung finden, hat man in der professionellen medizinischen Therapie erkannt, dass sich der Heilerfolg solcher Maßnahmen bedeutend verbessert, wenn man den betroffenen Körperbereich mit einer bestimmten, meist konstanten, Temperatur versorgt. Eine solche therapeutische Maßnahme, die sehr erfolgreich bei der Behandlung von traumatisierten oder entzündeten Gelenken eingesetzt wird, ist z. B. eine gleichmäßige Temperierung auf 15° C.

Um diese Temperierung zu erreichen, wird auf die betroffenen Körperbereiche eine Temperier-Manschette aufgelegt, die eine Vielzahl von Strömungswegen für Wasser enthält. Diese Manschette wird dann von einem Temperiergerät der hier in Rede stehenden Art mit temperiertem Wasser versorgt, so dass die gewünschte Temperatur über die Behandlungszeit im Wesentlichen konstant gehalten bzw. an einen gewünschten Temperaturverlauf angepasst werden kann.

Die bekannten Temperiergeräte werden sowohl im klinischen Bereich als auch im privaten Bereich eingesetzt. Für die Privatanwendung erwirbt der Patient entweder ein solches Gerät selbst oder mietet es für die Zeitdauer der Therapie.

Die im Folgenden genannten Geräte aus dem Stand der Technik, welche statt einer Manschette einen Katheter zur Temperierung des Körpers eines Patienten verwenden, sind für den Einsatz im klinischen Bereich vorgesehen:
Ein Temperiergerät für einen Dauerkatheter zum Wärmeaustausch mit dem Körper eines Patienten ist aus der US 2005/0028551 A1 bekannt. Das Kühlsystem für den Dauerkatheter weist dabei ein Wärmetauscherbad zur Aufnahme einer Leitung auf, durch welche eine Salzlösung dem Katheter zugeführt und von diesem abgeführt wird.

Ein Gerät zur Steuerung oder Regelung der Temperatur und des Flusses eines Wärmetauscherfluids für einen transluminal einführbaren Wärmetauscherkatheter ist aus der EP 1 820 480 A1 bekannt.

Ein System zur Regelung der Temperatur eines Patienten mit einem primären Fluidkreislauf mit einem primären Fluidwärmetauscher und einem sekundären Fluidkreislauf mit einem sekundären Wärmetauscher ist aus der EP 2 929 862 A1 bekannt. Der sekundäre Fluidkreislauf weist dabei einen in den Patienten einführbaren Wärmetauscherkatheter auf.

Es zeigt sich nun, dass die bekannten Geräte gerade, wenn sie im häuslichen Bereich eingesetzt werden, wo sie von Laien bedient werden müssen, nicht immer mit der wünschenswerten Zuverlässigkeit arbeiten.

Die vorliegende Erfindung stellt sich deshalb die Aufgabe, die Zuverlässigkeit eines solchen medizinischen Temperiergerätes zu erhöhen.

Diese Aufgabe wird erfindungsgemäß durch das medizinische Temperiergerät gemäß Anspruch 1 gelöst. Zu bevorzugende Weiterbildungen der Erfindung sind Gegenstand der Unteransprüche.

Ein medizinisches Temperiergerät der vorliegend betrachteten Art hat wenigstens ein Gehäuse, wenigstens eine Fluidversorgungseinrichtung, durch welche ein Fluid in dem Gehäuse bereitgestellt wird, wenigstens eine im Gehäuse angeordnete Temperiereinrichtung, die das durch das Gehäuse strömende Fluid temperiert, wenigstens eine Fluidabströmungseinrichtung, durch welche das Fluid mit einer im Wesentlichen vorbestimmten Temperatur aus dem Gehäuse herausgeführt wird, und wenigstens eine im Gehäuse angeordnete Pumpeneinrichtung, welche das durch das Gehäuse strömende Fluid mit Druck beaufschlagt.

Unter einer Fluidversorgungseinrichtung wird dabei eine Einrichtung verstanden, die das Vorhandensein des Fluids während des Betriebs des Temperiergeräts in stets ausreichender Menge sicherstellt oder es dem Benutzer des Temperiergeräts ermöglicht, dies zu tun, insbesondere durch geeignete Mittel zur Bevorratung und/oder Nachfüllung des Fluids.

Unter einer Temperiereinrichtung wird eine Einrichtung zum Heizen und/oder Kühlen des Fluids verstanden, vorzugsweise durch Umwandlung von elektrischer Energie in Wärmeenergie bzw. durch Verwendung von elektrischer Energie zum Entziehen von Wärmeenergie und vorzugsweise mit einem Wärmetauscher zur Übertragung von erzeugter Wärme auf das Fluid bzw. zum Entziehen von Wärme aus dem Fluid.

Erfindungsgemäß ist die wenigstens eine Pumpeneinrichtung als Verdrängerpumpe ausgebildet, und es ist eine Druckbegrenzungseinrichtung vorgesehen, welche bewirkt, dass der Druck des aus dem Gehäuse strömenden Fluids innerhalb eines vorbestimmten Bereichs gehalten ist.

Die Verdrängerpumpe ist vorzugsweise als Zahnradpumpe ausgeführt. Derartige Pumpen lassen sich kompakt konstruieren, sind weitgehend wartungsfrei und kostengünstig herstellen.

Das Fluid ist vorzugsweise Wasser, insbesondere Leitungswasser, kann aber auch destilliertes Wasser, Wasser mit bestimmten Zusatzstoffen oder ein anderes Fluid als Wasser, insbesondere Öl, sein.

Die Erfindung wird vorliegend am Beispiel eines Temperiergerätes zur Verwendung mit einer wasserdurchströmten Temperier-Manschette zum Auflegen auf die betroffenen Körperbereiche des Patienten beschrieben. Dies stellt jedoch keine Einschränkung dar; das erfindungsgemäße medizinische Temperiergerät lässt sich auch für andere Anwendungen einsetzen, beispielsweise zur Verwendung mit einem Temperierkörper, welcher in eine Körperöffnung des Patienten eingeführt wird.

Die vorliegende Erfindung bedeutet eine Abkehr von den bisherigen Prinzipien derartiger Temperiergeräte, die mit Strömungspumpen, insbesondere mit Kreiselpumpen, arbeiten. Auch wenn derartige Strömungspumpen, die z. B. als Umwälzpumpen in Heizungen etc. eingesetzt werden, vielfältige Vorteile in der Anwendung haben, erweist sich ihre Fördercharakteristik bei Anwendungen für medizinische Temperiergeräte als nachteilig.

Um die Temperierung für den Patienten so angenehm wie möglich zu gestalten, müssen für die Zuleitung vom Temperiergerät zur Manschette relativ leichte Schläuche verwendet werden. Derartige Schläuche sind aber, zumal wenn sie von Laien im häuslichen Umfeld verwendet werden, der Gefahr ausgesetzt, dass sie abgeknickt, abgedrückt oder sonstwie im Strömungsquerschnitt vermindert werden.

Entsprechendes gilt auch für die Manschette. Um sie optimal an die Körperform anzupassen, muss die Manschette relativ biegsam gestaltet sein. Auch hier besteht die Gefahr, dass die Durchströmung verhindert wird.

Eine nach dem Verdrängerprinzip arbeitende Pumpe hat den grundsätzlichen Vorteil, dass - je nach Antriebsleistung - ein hoher Druck erzeugt werden kann. Wird die Strömung durch die Zuführschläuche oder durch die Manschette behindert, erhöht sich der Druck in der Zuleitung entsprechend, und es kann trotz der Strömungsbehinderung ausreichend Fluid in die Manschette geführt werden. Dadurch ist auch in diesen Fällen der gewünschte Therapieerfolg sichergestellt.

In einer bevorzugten Ausführung der Erfindung weist die Fluidversorgungseinrichtung eine Fluidzuströmungseinrichtung auf, durch welche das Fluid von außen in das Gehäuse eingeführt wird.

Dabei ist die Fluidzuströmungseinrichtung vorzugsweise zur Einführung wenigstens eines Teils des durch die Fluidabströmungseinrichtung aus dem Gehäuse herausgeführten Fluids in das Gehäuse vorgesehen, so dass sich ein wenigstens teilweiser Fluidkreislauf ergibt.

Vorzugsweise weist das durch die Fluidzuströmungseinrichtung in das Gehäuse eingeführte Fluid eine andere Temperatur, und zwar vorzugsweise eine geringere Temperatur (im Falle des Einsatzes des Temperiergerätes zur Wärmebehandlung) bzw. vorzugsweise eine höhere Temperatur (im Falle des Einsatzes des Temperiergerätes zur Kältebehandlung), und/oder einen anderen, vorzugsweise geringeren, Druck auf als das durch die Fluidabströmungseinrichtung aus dem Gehäuse herausgeführte Fluid.

Erfindungsgemäß weist die Fluidversorgungseinrichtung einen im Gehäuse angeordneten Fluidbehälter auf. Somit kann der Benutzer das Temperiergerät auf bequeme Weise bei der Inbetriebnahme mit Fluid befüllen.

In einer weiteren bevorzugten Ausführung der Erfindung ist die Fluidzuströmungseinrichtung mit dem Fluidbehälter fluidleitend verbunden. Auf diese Weise kann der Fluidbehälter in den Fluidkreislauf eingebunden werden und dabei als Puffer für die zirkulierende Fluidmenge dienen, wobei eine beispielsweise durch Leckagen oder Verdunstung verloren gegangene Fluidmenge automatisch ausgeglichen wird.

Die Erfindung schlägt weiterhin vor, die vorgesehene Verdrängerpumpe mit einer Druckbegrenzungseinrichtung zu kombinieren, welche bewirkt, dass der Druck des aus dem Gehäuse strömenden Fluids innerhalb eines vorbestimmten Bereichs gehalten ist.

Dadurch wird verhindert, dass der Druck in den Zuführleitungen und/oder in der Manschette soweit ansteigt, dass die Verbindungen zwischen Gehäuse und Zuführleitungen bzw. zwischen Zuführleitung und Manschette undicht werden oder sich sogar lösen, und es wird verhindert, dass die Zuführleitungen und/oder die Manschette durch einen zu hohen Druck beschädigt werden können.

Es ist üblich, derartige Druckbegrenzungseinrichtungen als Druckbegrenzungsventile zu gestalten. Dabei wird z. B. eine Kugel über eine Feder in einen Verschlussbereich eines Strömungskanals gedrückt. Wird der Druck zu hoch, wird die Feder entsprechend weiter komprimiert und die Kugel von ihrem Sitz abgehoben, und das Fluid kann durch diesen Strömungskanal durchtreten.

Eine solche typische Druckbegrenzungseinrichtung kann auch im Zusammenhang mit der vorliegenden Erfindung verwendet werden. Die Erfindung schlägt jedoch eine anders gestaltete Druckbegrenzungseinrichtung vor, die besonders zuverlässig arbeitet und einfach gestaltet werden kann.

Eine solche vorteilhafte Druckbegrenzungseinrichtung ist erfindungsgemäß als Drosseleinrichtung gestaltet, die einen bestimmten höheren Strömungswiderstand aufweist. Sobald der Druck im System ansteigt, erhöht sich der Volumenstrom durch die Druckbegrenzungseinrichtung und baut den Druck entsprechend ab.

Erfindungsgemäß weist die Drosseleinrichtung eine Drosselleitung auf, welche an einem Ende mit der Pumpeneinrichtung fluidleitend verbunden ist. Die Drosselleitung ist vorzugsweise parallel zur Pumpeneinrichtung, d. h. als sogenannter "Bypass", geschaltet.

An ihrem der Pumpeneinrichtung abgewandten Ende ist die Drosselleitung erfindungsgemäß mit der Fluidversorgungseinrichtung, genauer mit derem Fluidbehälter, fluidleitend verbunden. Dadurch verbleibt sowohl das zum Druckausgleich durch die Drosselleitung strömende Fluid als auch ein Leckfluid, welches dadurch entsteht, dass eine solche Druckbegrenzungseinrichtung auch im normalen Druckbereich geringe Fluidmengen durchtreten lässt, innerhalb des Strömungskreislaufs.

Erfindungsgemäß besteht die fluidleitende Verbindung der Drosselleitung mit der Fluidversorgungseinrichtung im Wesentlichen aus einer Durchgangsbohrung in einer Wand des Fluidbehälters. Eine solche Durchgangsbohrung ist einfach und kostengünstig herzustellen.

Dabei ist der Innendurchmesser der Durchgangsbohrung vorzugsweise kleiner als der Innendurchmesser der Drosselleitung, vorzugsweise höchstens halb so groß, weiter vorzugsweise höchstens ein Drittel so groß und noch weiter vorzugsweise höchstens ein Viertel so groß. Dadurch lässt sich die Stärke der Drosselwirkung ebenso einfach wie kostengünstig festlegen.

In einer weiteren bevorzugten Ausführung der Erfindung dient die Drosselleitung auch als Entlüftungsleitung für die Pumpeneinrichtung. Diese Funktion ist insbesondere in der Anlaufphase der als Verdrängerpumpe ausgeführten Pumpeneinrichtung von Vorteil, da die Kammern der Verdrängerpumpe während dieser Phase noch teilweise oder vollständig mit Luft gefüllt sein können, woraus eine verringerte oder gar keine Pumpleistung resultiert. Die Pumpleistung setzt erst mit der Abführung der Luft aus den Kammern der Verdrängerpumpe ein. Durch die ohnehin vorhandene Drosselleitung ist daher keine separate Entlüftungsleitung mehr notwendig.

Die Erfindung wird im Folgenden anhand von Ausführungsbeispielen unter Bezugnahme auf die Zeichnungen erläutert. Dabei zeigen:
- Fig. 1:: ein erfindungsgemäßes Temperiergerät von vorne;
- Fig. 2:: das erfindungsgemäße Temperiergerät aus Fig. 1 von der Seite;
- Fig. 3:: eine Schnittansicht des erfindungsgemäßen Temperiergerätes von der Seite entlang der Linie A-A in Fig. 1 sowie eine vergrößerte Darstellung des Details X;
- Fig. 4:: das erfindungsgemäße Temperiergerät aus Fig. 1 von oben.

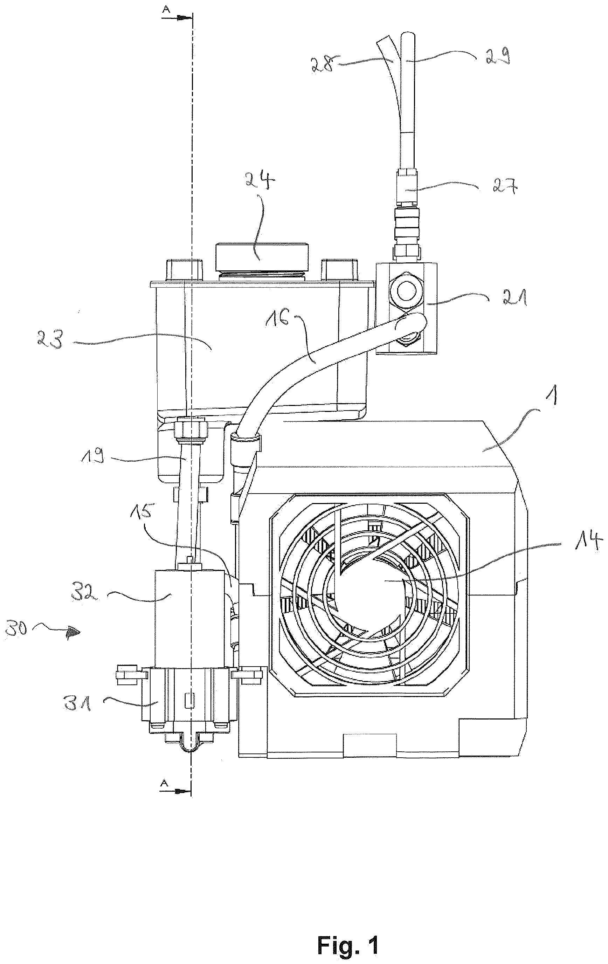

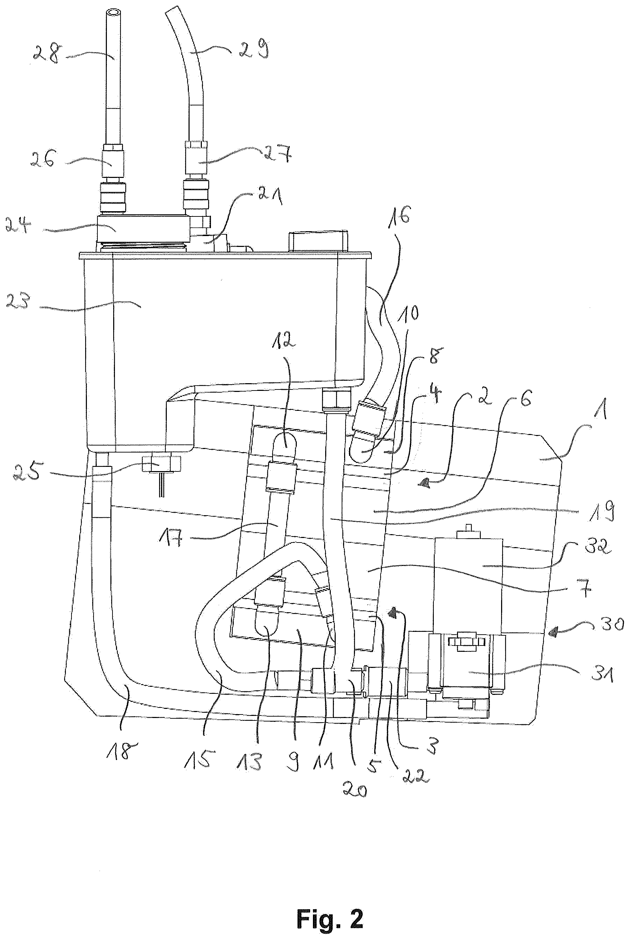

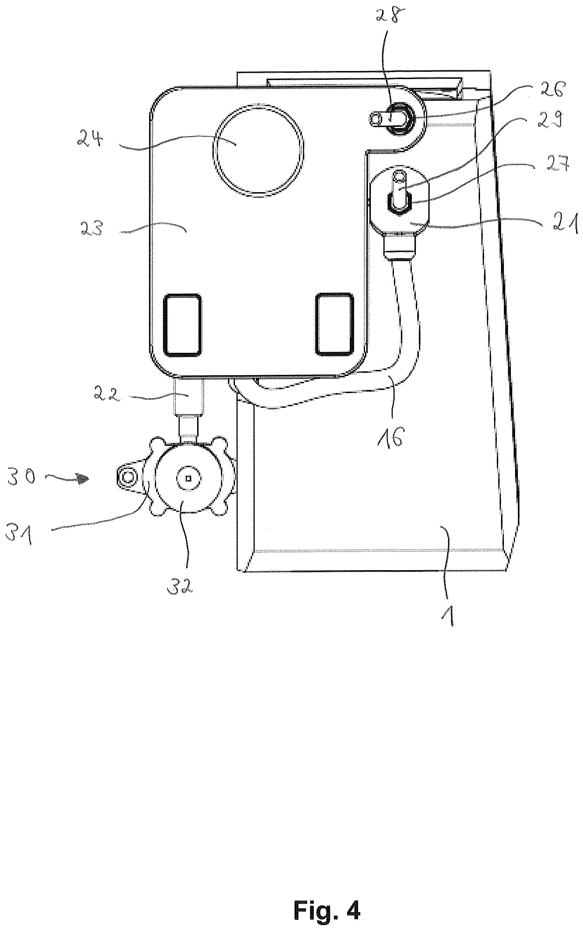

Die einzelnen Ansichten in den Fig. 1 bis 4 stellen jeweils dieselbe Ausführungsform des Temperiergerätes dar. Entsprechend bezeichnen gleiche Bezugszeichen in den Figuren auch gleiche Elemente dieser Ausführungsform. In der folgenden Beschreibung werden daher die Figuren nicht einzeln erläutert, sondern nur die in den Figuren dargestellte Ausführungsform als Ganzes.

Das erfindungsgemäße Temperiergerät weist als wesentliche Komponenten eine Pumpeneinheit 30, einen Fluidtank 23 sowie eine Temperiereinrichtung mit einer ersten Temperiereinheit 2 und einer zweiten Temperiereinheit 3 auf.

Die Pumpeneinheit 30 weist eine Zahnradpumpe 31, durch welche das Fluid mit Druck beaufschlagt wird, und einen elektrischen Pumpenmotor 32 zum Antrieb der Zahnradpumpe 31 auf. Der Pumpenmotor 32 wird vorzugsweise mit einer relativ geringen Spannung betrieben, im Ausführungsbeispiel etwa mit 5 V, im Gegensatz zu sonst bei Zahnradpumpen üblichen Betriebsspannungen von etwa 6 bis 14 V. Entsprechend wird die Zahnradpumpe 31 vorzugsweise mit einer relativ geringen Drehzahl betrieben, im Ausführungsbeispiel etwa mit 2570 min⁻¹, wodurch ein relativ hoher maximaler Fluiddruck erzeugt werden kann.

Die Zahnradpumpe 31 weist im Wesentlichen zwei über ein Untersetzungsgetriebe von dem Pumpenmotor 32 angetriebene Kunststoffzahnräder auf und ist dadurch preisgünstig herstellbar.

Die Temperiereinrichtung weist eine erste Temperiereinheit 2 und eine zweite Temperiereinheit 3 auf, welche von einem gemeinsamen Gehäuse 1 umschlossen sind. Das Gehäuse 1 kann dabei zweiteilig ausgeführt sein, damit die Temperiereinheiten 2, 3 in das Gehäuse 1 eingesetzt werden können.

Die beiden Temperiereinheiten 2, 3 sind als sogenannte "Liquid-to-Air-Module" ausgeführt. Im Ausführungsbeispiel sind die Temperiereinheiten 2, 3 baugleich. Sie können jedoch auch unterschiedlich gestaltet sein.

Die Temperiereinheiten 2, 3 weisen jeweils ein Peltier-Element 4, 5 auf, welches jeweils zwischen einem Kühlkörper 6, 7 und einem Wärmetauscher 8, 9 angeordnet ist und diese auf seinen gegenüberliegenden Seiten jeweils flächig berührt.

Durch die Peltier-Elemente 4, 5 wird im Betrieb des Temperiergerätes eine Temperaturdifferenz zwischen dem jeweiligen Wärmetauscher 8, 9 und dem jeweiligen Kühlkörper 6, 7 erzeugt, wodurch das Fluid, welches den Wärmetauscher 8, 9 durchströmt, je nach Anwendungsfall des Temperiergerätes gekühlt oder erwärmt wird. Über zwei Lüfter im Gehäuse 1, von denen lediglich ein Lüfter 14 in Fig. 1 dargestellt ist, wird die erwärmte Luft von den Kühlkörpern 6, 7 abgeführt bzw. den Kühlkörpern 6, 7 erwärmte Luft zugeführt, um die von den Peltier-Elementen 4, 5 erzeugte Temperaturdifferenz noch zu vergrößern. Im Ausführungsbeispiel beträgt die erzeugte Temperaturdifferenz etwa 11 Kelvin.

Der Fluidtank 23 dient der Einspeisung von Fluid in das Temperiergerät sowie zur Bevorratung von Fluid in dem Temperiergerät. Der Fluidtank 23 weist an seiner Oberseite eine Fluideinfüllöffnung 24 auf, durch die Fluid eingefüllt werden kann, sowie an seiner Unterseite eine Fluidablassöffnung 25, durch die - beispielsweise im Falle einer Stilllegung des Gerätes oder vor einem kompletten Austausch des Fluids - Fluid aus dem Fluidtank 23 abgelassen werden kann.

Der Fluidkreislauf während des Betriebs des erfindungsgemäßen Temperiergerätes verläuft vorzugsweise wie folgt:
Das von der Pumpeneinheit 30 kommende und dort mit Druck beaufschlagte Fluid fließt zunächst durch ein Verbindungsstück 22, durch welches die Zahnradpumpe 31 mit einem Verzweigungsstück 20 verbunden ist. Von dem Verzweigungsstück 20 zweigt eine Drosselleitung 19 ab, welche weiter unten noch näher beschrieben wird.

Der Fluidkreislauf setzt sich in einer Fluidleitung 15 fort, welche zu dem Anschluss 11 der zweiten Temperiereinheit 3 führt. Dort tritt das Fluid in den Wärmetauscher 9 der zweiten Temperiereinheit 3 ein, wird dort teilweise temperiert und tritt am Anschluss 13 wieder aus dem Wärmetauscher 9 aus. Danach fließt das Fluid über die Verbindungsleitung 17 zur ersten Temperiereinheit 2. Dort tritt das Fluid in den Wärmetauscher 8 der ersten Temperiereinheit 2 ein, wird dort ebenfalls teilweise temperiert und tritt am Anschluss 10 des Wärmetauschers 8 wieder aus. Damit ist das von der Pumpeneinrichtung 30 kommende Fluid vollständig temperiert.

Sodann fließt das Fluid durch die Fluidleitung 16 zu einem Verteiler 21, an welchen ein Schlauchanschlussstück 27 angeschlossen ist. An das Schlauchanschlussstück 27 kann ein Schlauch 29 angeschlossen werden. Dieser führt vorzugsweise zu einer Temperier-manschette zur Auflage auf einen Körperteil eines Patienten, insbesondere auf eine Hand, auf ein Knie oder auf einen anderen Körperteil, welcher temperiert werden soll. An diesen Körperteil wird Wärmeenergie abgegeben bzw. von diesem wird Wärmeenergie entzogen, je nachdem, ob eine Wärme- oder Kältebehandlung durchgeführt werden soll.

Eine an das Temperiergerät anzuschließende Manschette hat typischerweise einen Fluidinhalt von bis zu 0,5 Liter. Vorzugsweise wird lediglich eine Manschette an das Temperiergerät angeschlossen. Bei entsprechender Auslegung des Temperiergerätes können jedoch auch mehrere Manschetten angeschlossen werden. Zu diesem Zweck ist an dem Verteiler 21 noch ein Anschluss für ein weiteres Schlauchanschlussstück vorgesehen, welches im Ausführungsbeispiel jedoch nicht verwendet wird.

Von der Manschette fließt das Fluid vorzugsweise durch einen Schlauch 28, welcher durch ein Schlauchanschlussstück 26 mit dem Fluidtank 23 verbunden ist, in diesen zurück. Dabei hat das Fluid aufgrund des in der Manschette bewirkten Druckabfalls nur noch einen geringen Druck und aufgrund der Wärmeabgabe bzw. -aufnahme in der Manschette auch wieder annähernd seine Ausgangstemperatur. Je größer die Manschette ist, desto größer ist dabei im Allgemeinen auch der in der Manschette bewirkte Druckabfall und die Wärmeabgabe bzw. -aufnahme in der Manschette.

Das im Fluidtank 23 befindliche Fluid wird über eine weitere Fluidleitung 18, welche mit einer Öffnung in der Unterseite des Fluidtanks 23 verbunden ist, von der Zahnradpumpe 31 angesaugt, dort wieder mit Druck beaufschlagt, und der beschriebene Fluidkreislauf beginnt erneut. Selbstverständlich lassen sich die einzelnen, an dem Kreislauf beteiligten Einrichtungen auch in einer anderen Reihenfolge anordnen.

Für den Fall, dass die Fluidströmung in der Manschette behindert wird und dadurch der Druck im Temperiergerät und in der Manschette ansteigt, ist das Temperiergerät mit einer Drosselleitung 19, in welcher an wenigstens einer Stelle der Strömungsquerschnitt deutlich verringert ist, als Druckbegrenzungseinrichtung ausgestattet. Bei einem Druckanstieg im Fluidkreislauf erhöht sich der Volumenstrom durch die Drosselleitung 19 und baut derartige "Druckspitzen" oder auch einen über einen längeren Zeitraum erhöhten Druck entsprechend ab.

Im Ausführungsbeispiel wird der Fluiddruck typischerweise auf 0,5 bis 0,9 bar begrenzt, so dass auch bei höheren Fluidtemperaturen eine Verbrühungsgefahr weitgehend ausgeschlossen ist.

Die Drosselleitung 19 zweigt hinter der Zahnradpumpe 31 am Verzweigungsstück 20 von dem beschriebenen Fluidkreislauf ab und führt in Form eines "Bypass" direkt zum Fluidtank 23. Auf diese Weise fließt das zur Druckbegrenzung in die Drosselleitung 19 eingeleitete Fluid in den Fluidtank 23 und somit unmittelbar in den Fluidkreislauf zurück, wodurch Leckagen und damit ein Fluidverlust vermieden werden.

Die Stelle verringerten Strömungsquerschnitts in der Drosselleitung 19 hat vorzugsweise die Form einer Bohrung 35 geringen Durchmessers in der Unterseite des Fluidtanks 23, durch welche das Fluid am Ende der Drosselleitung 19 in den Fluidtank 23 hineinströmt. Im Ausführungsbeispiel hat die Bohrung 35 einen Durchmesser von etwa 1 mm, während die übrige Drosselleitung 19 einen Innendurchmesser von etwa 4 mm aufweist.

Die Bohrung 35 bildet auf einfache Weise einen hydraulischen Widerstand für das Fluid, und der Druck im Fluidkreislauf wird auf ebenso einfache Weise begrenzt.

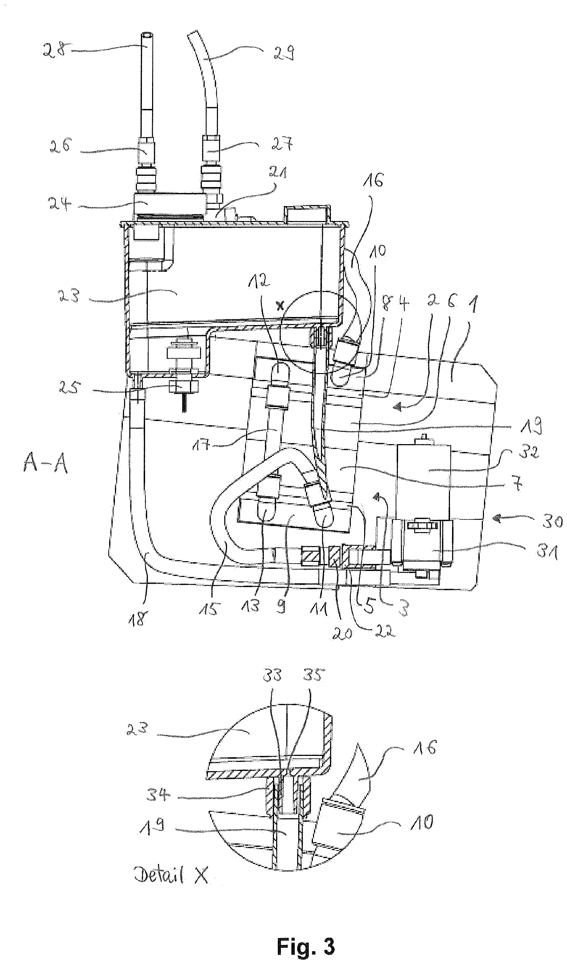

Wie besonders im Detail X in Fig. 3 zu erkennen, ist die Drosselleitung 19 auf einen an die Unterseite des Fluidtanks 23 angeformten Stutzen 33 aufgeschoben und durch eine Schlauchklemme 34 gesichert.

Weiterhin dient die Drosselleitung 19 als Entlüftung für die Zahnradpumpe 31.

Die Fluidleitungen 15 bis 18 sind vorzugsweise als Schläuche, insbesondere aus Polyurethan, ausgebildet, können aber auch starre Rohrleitungen, insbesondere aus Kunststoff oder Metall, sein.

### Bezugszeichenliste

- 1: Gehäuse
- 2: Erste Temperiereinheit
- 3: Zweite Temperiereinheit
- 4, 5: Peltier-Element
- 6, 7: Kühlkörper
- 8, 9: Wärmetauscher
- 10-13: Anschlüsse der Fluidleitungen
- 14: Lüfter
- 15-18: Fluidleitungen
- 19: Drosselleitung
- 20-22: Verbindungsstücke
- 23: Fluidtank
- 24: Fluideinfüllöffnung
- 25: Fluidablassöffnung
- 26,27: Schlauchanschlussstücke
- 28, 29: Schläuche
- 30: Pumpeneinheit
- 31: Zahnradpumpe
- 32: Pumpenmotor
- 33: Stutzen
- 34: Schlauchklemme
- 35: Bohrung

## Patentansprüche

1. Medizinisches Temperiergerät mit
wenigstens einem Gehäuse,
wenigstens einer Fluidversorgungseinrichtung, durch welche ein Fluid in dem Gehäuse bereitgestellt wird,
wenigstens einer im Gehäuse angeordneten Temperiereinrichtung (2, 3), die das durch das Gehäuse strömende Fluid temperiert,
wenigstens einer Fluidabströmungseinrichtung (27), durch welche das Fluid mit einer im Wesentlichen vorbestimmten Temperatur aus dem Gehäuse herausgeführt wird, und
wenigstens einer im Gehäuse angeordneten Pumpeneinrichtung (31), welche das durch das Gehäuse strömende Fluid mit Druck beaufschlagt,
wobei diese wenigstens eine Pumpeneinrichtung (31) als Verdrängerpumpe, vorzugsweise als Zahnradpumpe, ausgebildet ist und wobei eine Druckbegrenzungseinrichtung (19, 35) vorgesehen ist, welche bewirkt, dass der Druck des aus dem Gehäuse strömenden Fluids innerhalb eines vorbestimmten Bereichs gehalten ist, wobei die Druckbegrenzungseinrichtung (19, 35) eine Drosseleinrichtung ist und die Fluidversorgungseinrichtung einen im Gehäuse angeordneten Fluidbehälter (23) aufweist,
**dadurch gekennzeichnet,**
**dass** die Drosseleinrichtung eine Drosselleitung (19) aufweist, welche an einem Ende mit der Pumpeneinrichtung (31) und an dem der Pumpeneinrichtung (31) abgewandten Ende mit der Fluidversorgungseinrichtung fluidleitend verbunden ist, und dass die fluidleitende Verbindung der Drosselleitung (19) mit der Fluidversorgungseinrichtung im Wesentlichen aus einer Durchgangsbohrung (35) in einer Wand des Fluidbehälters (23) besteht.

2. Medizinisches Temperiergerät gemäß Anspruch 1, **dadurch gekennzeichnet, dass** die Fluidversorgungseinrichtung eine Fluidzuströmungseinrichtung (26) aufweist, durch welche das Fluid von außen in das Gehäuse eingeführt wird.

3. Medizinisches Temperiergerät gemäß Anspruch 2, **dadurch gekennzeichnet, dass** die Fluidzuströmungseinrichtung (26) zur Einführung wenigstens eines Teils des durch die Fluidabströmungseinrichtung (27) aus dem Gehäuse herausgeführten Fluids in das Gehäuse vorgesehen ist, so dass sich ein wenigstens teilweiser Fluidkreislauf ergibt, wobei das durch die Fluidzuströmungseinrichtung (26) in das Gehäuse eingeführte Fluid eine andere, vorzugsweise geringere, weiter vorzugsweise höhere, Temperatur und/oder einen anderen, vorzugsweise geringeren, Druck aufweist als das durch die Fluidabströmungseinrichtung (27) aus dem Gehäuse herausgeführte Fluid.

4. Medizinisches Temperiergerät gemäß Anspruch 2, **dadurch gekennzeichnet, dass** die Fluidzuströmungseinrichtung mit dem Fluidbehälter (23) fluidleitend verbunden ist.

5. Medizinisches Temperiergerät gemäß einem der vorhergehenden Ansprüche,
**dadurch gekennzeichnet, dass** der Innendurchmesser der Durchgangsbohrung (35) kleiner ist als der Innendurchmesser der Drosselleitung (19), vorzugsweise höchstens halb so groß, weiter vorzugsweise höchstens ein Drittel so groß und noch weiter vorzugsweise höchstens ein Viertel so groß.

6. Medizinisches Temperiergerät gemäß einem der vorhergehenden Ansprüche,
**dadurch gekennzeichnet, dass** die Drosselleitung (19) auch als Entlüftungsleitung für die Pumpeneinrichtung (31) dient.

## Claims

1. A medical temperature control apparatus comprising
at least one housing,
at least one fluid supply device by means of which a fluid is provided in the housing, at least one temperature control device (2, 3) which is arranged in the housing and which controls the temperature of the fluid flowing through the housing,
at least one fluid outflow device (27) through which the fluid is led out of the housing at a substantially predetermined temperature, and
at least one pump device (31) which is arranged in the housing and which causes the fluid flowing through the housing to be subjected to pressure,
wherein said at least one pump device (31) is constructed as a positive displacement pump, preferably as a gear pump, and wherein a pressure limiting device (19, 35) is provided which causes the pressure of the fluid flowing out of the housing to be kept within a predetermined range,
wherein the pressure limiting device (19, 35) is a throttle device and the fluid supply device comprises a fluid container (23) which is arranged in the housing,
**characterised in that**
the throttle device has a throttle line (19) which, at one end, is connected to the pump device (31) in a fluid-conducting manner, and which, at the end facing away from the pump device (31), is connected to the fluid supply device in a fluid-conducting manner, and **in that** the fluid-conducting connection of the throttle line (19) to the fluid supply device substantially consists of a through bore (35) in a wall of the fluid container (23).

2. The medical temperature control apparatus as claimed in claim 1, **characterised in that** the fluid supply device comprises a fluid inflow device (26) through which the fluid is introduced into the housing from the outside.

3. The medical temperature control apparatus as claimed in claim 2, **characterised in that** the fluid inflow device (26) is provided for introducing, into the housing, at least a portion of the fluid that is led out of the housing by the fluid outflow device (27), so that, at least partially, a fluid circuit results, wherein the fluid which is introduced into the housing through the fluid inflow device (26) has a different temperature, preferably a lower temperature, further preferably a higher temperature, and / or a different pressure, preferably a lower pressure, than the fluid which is led out of the housing through the fluid outflow device (27).

4. The medical temperature control apparatus as claimed in claim 2, **characterised in that** the fluid inflow device is connected to the fluid container (23) in a fluid-conducting manner.

5. The medical temperature control apparatus as claimed in any one of the preceding claims, **characterised in that** the inner diameter of the through bore (35) is smaller than the inner diameter of the throttle line (19), preferably at most half as large, further preferably at most one third as large and still further preferably at most one quarter as large as the inner diameter of the throttle line (19).

6. The medical temperature control apparatus as claimed in any one of the preceding claims, **characterised in that** the throttle line (19) also serves as a vent line for the pump device (31).

## Revendications

1. Appareil médical de thermorégulation avec au moins un boîtier,
au moins un dispositif d'alimentation en fluide, par lequel un fluide est fourni dans le boîtier,
au moins un dispositif de thermorégulation (2, 3) disposé dans le boîtier, qui thermorégule le fluide circulant à travers le boîtier,
au moins un dispositif d'évacuation de fluide (27), par lequel le fluide est sorti du boîtier à une température sensiblement prédéterminée, et
au moins un dispositif de pompe (31) disposé dans le boîtier, qui soumet le fluide circulant à travers le boîtier à l'action d'une pression,
dans lequel l'au moins un dispositif de pompe (31) est réalisé en tant que pompe volumétrique, de préférence en tant que pompe à engrenages, et dans lequel un dispositif de limitation de pression (19, 35) est prévu, lequel a pour effet que la pression du fluide sortant du boîtier est maintenue à l'intérieur d'une plage prédéterminée, dans lequel le dispositif de limitation de pression (19, 35) est un dispositif d'étranglement et le dispositif d'alimentation en fluide présente un réservoir de fluide (23) disposé dans le boîtier,
**caractérisé en ce**
**que** le dispositif d'étranglement présente une conduite d'étranglement (19), qui est reliée en écoulement de fluide sur une extrémité au dispositif de pompe (31) et sur l'extrémité opposée au dispositif de pompe (31) au dispositif d'alimentation en fluide, et que la liaison à écoulement de fluide de la conduite d'étranglement (19) au dispositif d'alimentation en fluide est constituée sensiblement d'un alésage de passage (35) dans une paroi du réservoir de fluide (23).

2. Appareil médical de thermorégulation selon la revendication 1, **caractérisé en ce que** le dispositif d'alimentation en fluide présente un dispositif d'arrivée de fluide (26), par lequel le fluide est introduit dans le boîtier depuis l'extérieur.

3. Appareil médical de thermorégulation selon la revendication 2, **caractérisé en ce que** le dispositif d'arrivée de fluide (26) est prévu pour introduire dans le boîtier au moins une partie du fluide sortant du boîtier par le dispositif d'évacuation de fluide (27), de sorte qu'il en résulte un circuit de fluide au moins partiel, dans lequel le fluide introduit dans le boîtier par le dispositif d'arrivée de fluide (26) présente une autre température, de préférence inférieure, de préférence plus élevée et/ou une autre pression, de préférence inférieure, que le fluide sorti du carter par le dispositif d'évacuation de fluide (27).

4. Appareil médical de thermorégulation selon la revendication 2, **caractérisé en ce que** le dispositif d'arrivée de fluide est relié en écoulement de fluide au réservoir de fluide (23).

5. Appareil médical de thermorégulation selon l'une quelconque des revendications précédentes, **caractérisé en ce que** le diamètre intérieur de l'alésage de passage (35) est inférieur au diamètre intérieur de la conduite d'étranglement (19), de préférence est égal au maximum à la moitié, par ailleurs de préférence au maximum à un tiers et plus encore de préférence au maximum à un quart de celui-ci.

6. Appareil médical de thermorégulation selon l'une des revendications précédentes, **caractérisé en ce que** la conduite d'étranglement (19) sert également de conduite d'évacuation d'air pour le dispositif de pompe (31).
